(19) Europäisches Patentamt

European Patent Office

Office européen des brevets

(11) Veröffentlichungsnummer: **0 408 075 B1**

## (12) EUROPÄISCHE PATENTSCHRIFT

(45) Veröffentlichungstag der Patentschrift: **08.03.95**

(51) Int. Cl.⁶: **C12Q 1/56**

(21) Anmeldenummer: **90113483.3**

(22) Anmeldetag: **13.07.90**

(54) **Verfahren und Reagenz zur Bestimmung von Antithrombin III.**

(30) Priorität: **14.07.89 DE 3923340**

(43) Veröffentlichungstag der Anmeldung:
**16.01.91 Patentblatt 91/03**

(45) Bekanntmachung des Hinweises auf die
Patenterteilung:
**08.03.95 Patentblatt 95/10**

(84) Benannte Vertragsstaaten:
**AT BE CH DE DK ES FR GB GR IT LI LU NL SE**

(56) Entgegenhaltungen:
**EP-A- 0 034 320**
**EP-A- 0 103 247**
**EP-A- 0 110 306**
**EP-A- 0 336 353**

**H. LILL et al., "Anti Thrombin III. Heparin Cofactor", Band V, 3. Aufl., Verlag Chemie, Weinheim (DE); Seiten 441-448&NUM;**

**ANALYTICAL BIOCHEMISTRY Band 144, 1985, Seiten 165-171, New York, NY (US); C.C. MIRAGLIA et al., Seiten 165-171&NUM;**

(73) Patentinhaber: **BOEHRINGER MANNHEIM GMBH**

**D-68298 Mannheim (DE)**

(72) Erfinder: **Dessauer, Andreas, Dr.**
**Herre-Strasse 1**
**D-8132 Tutzing (DE)**
Erfinder: **Herz, Reinhard**
**Kurt Stieler-Strasse 1**
**D-8134 Pöcking-Possenhofen (DE)**
Erfinder: **Lill, Helmut, Dr.**
**Zugspitzstrasse 24**
**D-8121 Wielenbach (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

EP 0 408 075 B1

**Beschreibung**

Die Erfindung betrifft ein Verfahren zur Bestimmung von Antithrombin III in Körperflüssigkeiten durch Umsetzung der Probe mit Thrombin und einem chromogenen Substrat, das unter Einwirkung von Thrombin eine Farbe bildet und Messung der entstehenden Farbe sowie ein dazu geeignetes Reagenz.

Antithrombin III ist ein Faktor des Blutgerinnungssystems, der der Regulation dient. Die Blutgerinnung wird in Gang gesetzt durch das kaskadenartige Zusammenwirken verschiedener Proteasen. Der letzte der hintereinandergeschalteten Aktivierungsschritte setzt Thrombin frei, das wiederum durch Spaltung von Fibrinogen Fibrinmonomere erzeugt, die sich zusammenlagern und einen Thrombus bilden. Wichtigster Regulator ist Antithrombin III, (AT III) das mit Thrombin und auch mit anderen an der Blutgerinnung beteiligten Proteasen einen Komplex bilden kann, der das aktive Zentrum blockiert. Der Antithrombin III-Gehalt im Blut liegt bei gesunden Menschen in einem relativ engen Bereich. Verminderte Antithrombin III-Gehalte können durch eine Verbrauchskoagulopathie, eine schwere Lebererkrankung oder erblich bedingt sein. Ein verminderter Antithrombin III-Gehalt wird heute allgemein als Thromboserisiko gewertet. Deshalb ist in manchen Fällen der Antithrombin III-Gehalt auch bei einer akuten Thrombose reduziert. Der Antithrombin III-Gehalt ist daher ein wertvoller Parameter in der klinischen Diagnostik.

Es sind bereits verschiedene Verfahren zum Nachweis von Antithrombin III bekannt, sowohl auf Basis immunologischer Methoden als auch unter Verwendung chromogener Substrate. Im letzteren Fall wird der Probe Thrombin zugesetzt, das mit in der Probe vorhandenem AT III reagiert und inaktiviert wird. Überschüssiges Thrombin wird dann durch Inkubation mit einem chromogenen Substrat, das durch Einwirkung von Thrombin eine gefärbte Substanz bildet, und Auswertung der Farbbildung nachgewiesen, wobei der Antithrombin III-Gehalt in indirektem Verhältnis zu der Farbbildung steht. Verfahren zur Bestimmung von Antithrombin III sind beispielsweise beschrieben in Bergmeyer, "Methods of Enzymatic Analysis", 3d edition, Verlag Chemie, Vol.5, S. 441-448; I. Witt, ed., "Neue Methoden der Gerinnungsanalyse mit chromogenen Substraten", Stormorken, Neue Methoden der Gerinnungsanalyse, Seite 119-121; Odegard et al., Haemostasis 7: 202-209 (1978); Fareed et al. in Chromogenic Peptide Substrates (eds. M.F. Scully and V.V. Kakkar) Churchill Livingstone (1979) 183-191 und Abildgaard et al. Thromb. Res. 11, 549-553 (1977).

In der Regel werden, um Störungen im Test zu vermeiden, die Proben sehr stark verdünnt, da Eigenfarbe, Trübung und Abweichungen im pH-Wert sowie Salzgehalt der Probe das Meßergebnis umso weniger beeinflussen, je kleiner die Probenmenge im Testansatz bemessen werden kann. Bei Durchführung kinetischer Bestimmungen insbesondere an Analyseautomaten wird daher mit Probenverdünnungen im Bereich von bis zu 1:1500 gearbeitet. Dies ist jedoch für die Routine (speziell Notfallsituation) unpraktisch.

Es war daher Aufgabe der Erfindung, bekannte Nachweismethoden zu verbessern und ein Verfahren zur Verfügung zu stellen, das in Analyseautomaten durchgeführt werden kann, in kurzer Zeit und ohne Probenverdünnung durchführbar ist und den Einsatz eines einzigen Standards ermöglicht.

Diese Aufgabe wird gelöst durch ein Verfahren zur Bestimmung von Antithrombin III in Körperflüssigkeiten durch Umsetzung der Probe mit Thrombin und einem chromogenen Substrat, das unter Einwirkung von Thrombin eine Farbe bildet und Messung der entstehenden Farbe, das dadurch gekennzeichnet ist, daß man die Umsetzung in Gegenwart von denaturierenden Agentien oder von Tetrapeptid Gly-Pro-Arg-Pro durchführt und daß man als Substrat ein Oligopeptidsubstrat verwendet, dessen Empfindlichkeit gegenüber Thrombin, verglichen mit Tos-Gly-Pro-Arg-pNA, um den Faktor 5 bis 100 geringer ist.

Überraschenderweise gelingt es durch Zusatz von denaturierenden Agentien bzw. eines Tetrapeptids und durch Einsatz eines relativ unempfindlichen Oligopeptid-Substrates für Thrombin, Antithrombin III genau und reproduzierbar zu bestimmen, wobei auch längere Testserien ohne Beeinträchtigung am Analyseautomaten durchgeführt werden können.

Zur Bestimmung von Antithrombin III in einer Körperflüssigkeit wird eine Probelösung mit Thrombin im Überschuß (bezogen auf Antithrombin III) versetzt. Dabei bilden sich aus Antithrombin III und Thrombin Komplexe. Derartig komplexiertes Thrombin hat keine Protease-Aktivität mehr. Zu der Lösung wird dann ein Substrat, das von Thrombin unter Farbbildung gespalten werden kann, zugegeben. Nur das im Überschuß vorhandene, nicht komplexierte Thrombin reagiert mit dem Substrat. Das gemessene Signal ist umgekehrt proportional zur Antithrombin III-Konzentration der Probe. Bevorzugt wird zur Durchführung des Verfahrens Heparin zugesetzt, das die Reaktion zwischen Antithrombin III und Thrombin beschleunigt.

Es gibt eine Vielzahl von für dieses Verfahren geeigneten Substraten, von denen das am meisten verwendete Tos-Gly-Pro-Arg-pNa-Acetat (Chromozym[R] TH) ist. Dieses Reagenz ist hochempfindlich.

Erfindungsgemäß wird nun zusätzlich im ersten Schritt ein denaturierendes Agens oder das Tetrapeptid Gly-Pro-Arg-Pro zugesetzt. Es wurde gefunden, daß bei längeren Testserien der Thrombinausgangswert bei Verwendung unverdünnter Proben nicht konstant bleibt. Dies wird durch Zusatz der obengenannten Komponenten behoben. Geeignet als denaturierende Agentien sind die an sich auf diesem Gebiet bekannten Substanzen. Bevorzugt wird Harnstoff oder Guanidiniumhydrochlorid verwendet. Das denaturierende Agens wird bevorzugt in einer Konzentration von 0,1 bis 1 mol/l im Test bzw. 1 bis 6 mol/l in der Substratlösung eingesetzt. Wenn als Komponente das Tetrapeptid verwendet wird, so wird dieses bevorzugt in einer Konzentration im Bereich von 0,04 bis 1 mg/ml im Test zugesetzt.

Das denaturierende Agens bzw. das Tetrapeptid kann entweder bereits der Reagenzlösung zugesetzt werden, oder aber bei Inkubation der Probelösung mit der Reagenzlösung zugesetzt werden. Wenn Harnstoff als denaturierendes Agens verwendet wird, so ist es vorteilhaft, den Harnstoff erst zusammen mit dem Substrat zuzusetzen, da Harnstoff auf die Stabilität des Thrombins einen ungünstigen Einfluß hat.

Weiteres wesentliches Merkmal des erfindungsgemäßen Verfahrens ist die Verwendung eines chromogenen Oligopeptidsubstrats für Thrombin, dessen Empfindlichkeit aber verglichen mit Chromozyn[R] TH um den Faktor 5 bis 100 geringer ist. Alle Substrate, die diese Bedingung erfüllen, sind geeignet, wie z.B.

CBZ-Val-Gly-Arg-pNA;

H-D-Pro-Phe-Arg-pNA;

H-D-Val-Leu-Arg-pNA;

Bz-Val-Leu-Arg-pNA;

Bz-Leu-Leu Arg-pNA;

Bz-Phe-Leu-Arg-pNA sowie

Bz-Leu-Ile -Arg-pNA. (CBZ: Carbobenzoxy, Bz: Benzoyl, D-Pro: D-Prolin, D-Val: D-Valin, pNA: p-Nitroanilin). Diese als Beispiel genannten Verbindungen tragen als Chromophor alle p-Nitroanilin, das von Thrombin abgespalten wird. Andere übliche Chromophore sind ebenso geeignet wie z.B. 5-Amino-2-nitrobenzoesäure oder Methoxy-nitroanilin, die anstelle von pNA in dem Substrat vorhanden sein können. Bevorzugt wird als Substrat ein Peptid der Formel R-OCO-Gly-Pro-Arg-pNA eingesetzt, worin R einen Alkylrest mit 1 bis 3 C-Atomen bedeutet und bevorzugt ein Methylrest ist.

Besonders bevorzugt werden Substrate eingesetzt, deren Empfindlichkeit um den Faktor 10 bis 30 gegenüber Chromozym® TH vermindert ist.

Die Konzentration des Substrats in der das Substrat in der Testlösung vorhanden ist, richtet sich nach seiner Michaelis-Konstante. Geeignet sind Substrat-Konzentrationen im Bereich von dem 2- bis 20-fachen der jeweiligen Michaelis-Konstante.

Die für das Verfahren eingesetzte Thrombin-Konzentration kann erfindungsgemäß bis zum 30fachen, bevorzugt 7- bis 15fachen der nach dem Stand der Technik verwendeten Thrombin-Konzentration, entsprechend bis zu etwa 630 U Thrombin/l (Internationale Enzymeinheiten Thrombin mit Tos-Gly-Pro-Arg-pNA als Substrat bei 25°C) Testlösung, erhöht sein. Innerhalb dieses Bereichs, jedoch nicht mit weniger Thrombin, kann mit dem erfindungsgemäßen Verfahren noch eine lineare Eichkurve erhalten werden, so daß die Durchführung einer kinetischen Bestimmung in einem weiten Bereich und vor allem eine komfortable Testführung mit nur einem Standard am Analysenautomaten möglich wird. Ist nämlich die Linearität über den Meßbereich nicht gegeben, dann muß mit mehreren Standards gearbeitet werden, was die schnelle Anwendung des Tests, die z.B. für Notsituationen essentiell ist, erschwert.

Die Probenmenge richtet sich nach dem Meßbereich des Testes sowie nach den technischen Möglichkeiten. Die untere Grenze liegt für Analysenautomaten bei 1 µl. Die obere Grenze ist vom Typ des Analysenautomaten abhängig und beträgt üblicherweise nicht mehr als 5 µl. Ist die Probenmenge höher, steht unter sonst gleichen Testbedingungen ein geringerer Meßbereich zur Verfügung, außerdem treten bei erhöhter Probenmenge in zunehmendem Maße Störungen durch Fibrinbildung auf. Andererseits reduziert sich das eigentliche Meßsignal bei kleinerer Probenmenge, was mit der steigenden Pipettierungenauigkeit sehr kleiner Volumina zu einer Verschlechterung der Präzision des Tests führt.

Ein weiterer Gegenstand der Erfindung ist ein Reagenz zur Bestimmung von Antithrombin III, das Thrombin, ein denaturierendes Agens oder das Tetrapeptid Gly-Pro-Arg-Pro sowie ein chromogenes Substrat, das unter Einwirkung von Thrombin eine Farbe bildet und verglichen mit Chromozym® TH, bezogen auf Thrombin, eine um den Faktor 5 bis 100 geringere Empfindlichkeit hat, enthält.

In einer bevorzugten Ausführungsform enthält das Reagenz zusätzlich noch Heparin.

Die Erfindung wird durch die folgenden Beispiele erläutert:

Beispiel 1 (Vergleich)

(Verfahren nach Stand der Technik, Bergmeyer)

1 Teil Thrombin (500 U/l) wird mit 20 Teilen Tris/HCl-Puffer, pH 8,1, der gleichzeitig Heparin in ausreichender Menge (2 USP-Einheiten/ml) enthält, gemischt. Zur Messung des Thrombinausgangswerts werden 2 ml dieses Thrombinreagenzes mit 0,10 ml physiologischer Kochsalzlösung und zum Start der Enzym/Substrat-Reaktion mit 0,200 ml Thrombinsubstratlösung (Chromozym® TH: Tos-Gly-Pro-Arg-pNA, 0,16 mmol/l im Testansatz) versetzt. In dem so definierten Testansatz werden Reaktionsgeschwindigkeiten (ΔE/min) bei 405 nm bei 25°C von 0,220 und bei 37°C von 0,400 erreicht.

Beispiel 2

Es wurde Antithrombin III in Probelösungen nach dem im Beispiel 1 beschriebenen Verfahren bestimmt, wobei jedoch Chromozym® TH durch die erfindungsgemäßen Substrate ersetzt wurde.
Manueller Test ohne Probenvorverdünnung:
0,01 ml unverdünnte Probe (entspr. 6,6 µl Probe pro ml Testvol.)
1,25 ml Thrombinreagenz (308 U/l Thrombin im Test, 14,74-fach gegenüber Stand der Technik)
0,25 ml Substrat (0,298 mmol/l Methyl-OCO-Gly-Pro-Arg-pNA, 0,5 mol/l Harnstoff im Test.

Tests an Analysenautomaten:
- Hitachi 704/705:
  0,003 ml unverdünnte Probe (entspr. 7,1 µl Probe pro ml Testvol.)
  0,350 ml Thrombinreagenz (308 U/l Thrombin und 248 mg/l Gly-Pro-Arg-Pro im Test)
  0,070 ml Substrat (0,298 mmol/l MeOCO-Gly-Pro-Arg-pNA im Test)
- Hitachi 717:
  0,002 ml unverdünnte Probe (entspr. 6,6 µl Probe pro ml Testvol.)
  0,250 ml Thrombinreagenz (308 U/l Thrombin und 248 mg/l Gly-Pro-Arg-Pro im Test)
  0,050 ml Substrat (0,298 mmol/l MeOCO-Gly-Pro-Arg-pNA im Test)

## Tabelle I

Vergleich der Thrombinausgangswerte (ohne Probe) unter den hier gewählten Testbedingungen bei Verwendung von Chromozym® TH und MeOCO-Gly-Pro-Arg-pNA:

|  | MeOCO-Gly.... (ΔE/min) | Chromozym®TH (ΔE/min) |
|---|---|---|
| Hitachi 717, 37°C | 0,400 | 5,700 |
| Hitachi 717, 25°C | 0,200 | 2,900 |
| Hitachi 704, 37°C | 0,300 | 4,300 |
| Hitachi 704, 25°C | 0,150 | 2,100 |

4

Für die Hitachigeräte liegt die Grenze der Meßbarkeit von kinetischen Enzym/Substratreaktionen bei ca. 1,00 ΔE/min. Auch die anderen bekannten Analysengeräte stoßen hier bei diesen hohen ΔE/min-Werte auf nicht mehr machbare analytische Grenzen. Das Gleiche gilt selbstverständlich für den manuellen Test, bei dem unter den oben skizzierten Bedingungen ohne Probenvorverdünnung mit Chromozym® TH als Substrat ΔE-Werte vom 3,3 bei 25°C und von 6,0 bei 37°C auftreten würden.

Tabelle II

Testgerät Hitachi 717

Testansatz: 0,002 ml unverdünnte Probe
0,250 ml Thrombinreagenz; 14,74-fach
(= 308 U/l im Test)
0,050 ml Substrat

Technik: verglichen mit Stand der Technik

| Substratsensitivität (% von Chromozym® TH) | Thrombinkonzentr. im Test | | | | | |
| --- | --- | --- | --- | --- | --- | --- |
| | 7,5-fach (156,8 U/l) 25°C | 7,5-fach 37°C | 14,74-fach (308 U/l) 25°C | 14,74-fach 37°C | 30-fach (627 U/l) 25°C | 30-fach 37°C |
| 1 % | 0,014 | 0,028 | 0,029 | 0,057 | 0,058 | 0,114 |
| 2 % | 0,029 | 0,057 | 0,058 | 0,114 | 0,116 | 0,228 |
| 7 % z.B.Me-OCO-Gly-Pro-Arg-pNA | 0,100 | 0,200 | 0,200 | 0,400 | 0,400 | 0,800 |
| 17 % z.B. i-Prop-OCO-Gly-Pro-Arg-pNA | 0,246 | 0,484 | 0,493 | 0,969 | 0,986 | 1,938 |
| 20 % | 0,280 | 0,560 | 0,580 | 1,140 | 1,160 | 2,280 |

Es wird deutlich, daß mit den Substraten höherer Sensitivität (17 % bzw. 20 % von Chromozym® TH) nicht alle Testversionen (z.B. 37°C bzw. höhere Thrombinkonz.) mit sehr guten Ergebnissen durchgeführt werden können; der Rahmen, innerhalb dessen das erfindungsgemäße Verfahren vorteilhaft ohne Probenverdünnung durchgeführt werden kann, ist ersichtlich.

**Patentansprüche**

1. Verfahren zur Bestimmung von Antithrombin III in Körperflüssigkeiten durch Umsetzung der Probe mit Thrombin und einem chromogenen Substrat, das unter Einwirkung von Thrombin eine Farbe bildet und Messung der entstehenden Farbe, **dadurch gekennzeichnet,** daß man die Umsetzung in Gegenwart von denaturierenden Agentien oder von Tetrapeptid Gly-Pro-Arg-Pro durchführt und daß man als Substrat ein Oligopeptidsubstrat verwendet, dessen Empfindlichkeit gegenüber Thrombin, verglichen mit Tos-Gly-Pro-Arg-pNA, um den Faktor 5 bis 100 geringer ist.

2. Verfahren nach Anspruch 1, **dadurch gekennzeichnet,** daß man als denaturierendes Agens Harnstoff oder Guanidiniumhydrochlorid verwendet.

3. Verfahren nach Anspruch 2, **dadurch gekennzeichnet,** daß man das denaturierende Agens in einer Konzentration von 0,1 bis 1 mol/l im Test verwendet.

4. Verfahren nach Anspruch 1, **dadurch gekennzeichnet,** daß man das Tetrapeptid in einer Konzentration von 0,04 bis 1 mg/ml im Test verwendet.

5. Verfahren nach einem der vorhergehenden Ansprüche, **dadurch gekennzeichnet,** daß man als Oligopeptidsubstrat ein Peptid mit der Formel
    R-OCO-Gly-Pro-Arg-pNA,
worin R ein Alkylrest mit 1 bis 3 C-Atomen ist, verwendet.

6. Verfahren nach einem der vorhergehenden Ansprüche, **dadurch gekennzeichnet,** daß man ein Oligopeptidsubstrat verwendet, dessen Empfindlichkeit verglichen mit Tos-Gly-Pro-Arg-pNA um den Faktor 10 bis 30 geringer ist.

7. Reagenz zur Bestimmung von Antithrombin III, **dadurch gekennzeichnet,** daß es Thrombin, ein denaturierendes Agens oder das Tetrapeptid Gly-Pro-Arg-Pro und als Substrat, das unter Einwirkung von Thrombin eine Farbe bildet, ein Oligopeptidsubstrat enthält, dessen Empfindlichkeit verglichen mit Tos-Gly-Pro-Arg-pNA um den Faktor 5 bis 100 vermindert ist.

**Claims**

1. Method for the determination of antithrombin III in body fluids by reacting the sample with thrombin and a chromogenic substrate which forms a colour by the action of thrombin and measurement of the colour formed, **wherein** the reaction is carried out in the presence of denaturing agents or of the tetrapeptide Gly-Pro-Arg-Pro and wherein an oligopeptide substrate is used as the substrate which has a sensitivity to thrombin which is a factor of 5 to 100 lower compared with Tos-Gly-Pro-Arg-pNA.

2. Method as claimed in claim 1, **wherein** urea or guanidinium hydrochloride is used as the denaturing agent.

3. Method as claimed in claim 2, **wherein** the denaturing agent is used in a concentration of 0.1 to 1 mol/l in the test.

4. Method as claimed in claim 1, **wherein** the tetrapeptide is used in a concentration of 0.04 to 1 mg/ml in the test.

5. Method as claimed in one of the previous claims, **wherein** a peptide having the formula
    R-OCO-Gly-Pro-Arg-pNA
in which R is an alkyl residue with 1 to 3 C atoms is used as the oligopeptide substrate.

6. Method as claimed in one of the previous claims, **wherein** an oligopeptide substrate is used which has a sensitivity which is a factor 10 to 30 lower compared with Tos-Gly-Pro-Arg-pNA.

7. Reagent for the determination of antithrombin III, **wherein** it contains thrombin, a denaturing agent or the tetrapeptide Gly-Pro-Arg-Pro and an oligopeptide substrate as substrate which forms a colour by the action of thrombin and which has a 5 to 100-fold lower sensitivity compared with Tos-Gly-Pro-Arg-pNA.

**Revendications**

1. Procédé de détermination de l'antithrombine III dans des liquides biologiques par réaction de l'échantillon avec la thrombine et un substrat chromogène qui forme une couleur sous l'action de la thrombine et mesure de la couleur formée, caractérisé en ce que l'on conduit la réaction en présence d'agents dénaturants ou du tétrapeptide Gly-Pro-Arg-Pro et en ce que l'on utilise comme substrat un substrat oligopeptidique dont la sensibilité vis à vis de la thrombine, comparée à celle de Tos-Gly-Pro-Arg-pNA, est plus faible d'un facteur 5 à 100.

2. Procédé selon la revendication 1, caractérisé en ce que l'on utilise comme agent dénaturant l'urée ou le chlorhydrate de guanidinium.

3. Procédé selon la revendication 2, caractérisé en ce que l'on utilise l'agent dénaturant en une concentration de 0,1 à 1 mol/l dans le test.

4. Procédé selon la revendication 1, caractérisé en ce que l'on utilise le tétrapeptide en une concentration de 0,04 à 1 mg/ml dans le test.

5. Procédé selon l'une des revendications précédentes, caractérisé en ce que l'on utilise comme substrat oligopeptidique un peptide de formule:
    R-OCO-Gly-Pro-Arg-pNA
dans laquelle R est un reste alkyle de 1 à 3 atomes de carbone.

6. Procédé selon l'une des revendications précédentes, caractérisé en ce que l'on utilise un substrat oligopeptidique dont la sensibilité, comparée à celle de Tos-Gly-Pro-Arg-pNA, est plus faible d'un facteur 10 à 30.

7. Réactif pour la détermination de l'antithrombine III, caractérisé en ce qu'il contient de la thrombine, un agent dénaturant ou le tétrapeptide Gly-Pro-Arg-Pro et comme substrat qui forme une couleur sous l'action de la thrombine un substrat oligopeptidique dont la sensibilité, comparée à celle de Tos-Gly-Pro-Arg-pNA, est réduite d'un facteur 5 à 100.